# FASCICULE DE BREVET EUROPEEN

(11) **EP 0 655 008 B1**
(45) Date de publication et mention de la délivrance du brevet: **19.11.1997**
(21) Numéro de dépôt: 94918412.1
(22) Date de dépôt: 06.06.1994
(51) Int. Cl.: A61N 5/10

(54) **DISPOSITIF DE TRAITEMENT DE LESIONS CEREBRALES PAR RAYONNEMENT GAMMA, ET APPAREIL DE TRAITEMENT CORRESPONDANT**
VORRICHTUNG ZUR BEHANDLUNG VON GEHIRNSCHÄDIGUNGEN DURCH GAMMASTRAHLUNG UND BEHANDLUNGSGERÄT
DEVICE FOR TREATING BRAIN DAMAGE BY GAMMA RADIATION, AND CORRESPONDING TREATMENT APPARATUS

(30) Priorité: 07.06.1993 FR 9306787
(43) Date de publication de la demande: 31.05.1995
(73) Titulaire: ATEA SOCIETE ATLANTIQUE DE TECHNIQUES AVANCEES, F-44470 Carquefou (FR)
(72) Inventeur: KOPECKY, Bernard, F-44300 Nantes (FR)
(74) Mandataire: Jacobson, Claude
(86) Numéro de dépôt international: FR9400667
(87) Numéro de publication internationale: WO9428973

(56) Documents cités:
- EP-A- 0 248 774
- EP-A- 0 371 303
- EP-A- 0 431 785
- FR-A- 2 672 220

## Description

La présente invention est relative à un dispositif de traitement de lésions cérébrales par rayonnement gamma, comprenant un ensemble sources-collimateur à peu près hémisphérique comportant un grand nombre de sources de rayons gamma associées à des canaux qui sont tous orientés vers un même point de focalisation.

Des appareils à rayonnement gamma ont été proposés pour la neurochirurgie non intrusive du cerveau, pour traiter les lésions cérébrales sans ouvrir la boîte crânienne. La destruction des lésions est obtenue par concentration précise d'un rayonnement gamma sur les zones à traiter telles que des malformations veineuses ou des tumeurs. Ces appareils sont souvent désignés sous le vocable "appareils de chirurgie gamma" ou "bistouris gamma".

Dans ces appareils, on dispose de sources externes de rayonnement gamma qu'il s'agit de diriger et de concentrer de façon précise sur la lésion traitée pour faire absorber par celle-ci la dose souhaitée sans léser notablement les tissus intermédiaires qui s'interposent entre les sources et la lésion, ni les tissus environnant la lésion et, d'une façon générale, en réduisant au minimum les doses de rayonnement absorbées par les tissus sains.

Ceci est obtenu notamment par les dispositifs du type précité, dont un exemple est décrit dans le FR-A-2 672 220. Dans cette technologie, on utilise ainsi un grand nombre de sources extérieures fixes, collimatées individuellement et disposées radialement de façon que les axes des faisceaux qu'elles produisent convergent en un point focal qui coïncide avec la lésion à traiter. L'intensité de chaque faisceau est insuffisante pour léser les tissus sains intermédiaires qu'il traverse, d'autant plus que le dispositif est animé, pendant le traitement, d'un mouvement angulaire autour du point de focalisation. En revanche, au point de convergence ou point focal, la dose reçue par la lésion est suffisante pour assurer sa destruction.

Dans cette technique connue, à chaque source est associé un canal unique au moins à peu près conique dont les génératrices convergent vers le point de focalisation et dans lequel est positionnée une aiguille centrale conique en matériau absorbant les rayons gamma.

L'invention a pour but de perfectionner ce dispositif connu de manière à mieux concentrer le rayonnement au point de focalisation, afin d'améliorer l'efficacité du traitement tout en protégeant mieux du rayonnement gamma les zones saines du cerveau.

A cet effet, l'invention a pour objet un dispositif de traitement du type précité, caractérisé en ce que chaque source de rayons gamma est associée à un faisceau de canaux inscrit dans une enveloppe de révolution conique dont le sommet est situé au point de focalisation.

Le dispositif peut comporter une ou plusieurs des caractéristiques suivantes :
- chaque source se trouve en regard de l'ouverture d'entrée de tous les canaux du faisceau qui lui est associé;
- tous les canaux sont cylindriques;
- tous les canaux ont sensiblement les mêmes dimensions;
- chaque faisceau comporte au moins cinq canaux.

L'invention a également pour objet un appareil de traitement de lésions cérébrales par rayonnement gamma comprenant un dispositif tel que défini ci-dessus, et une table porte-patient mobile équipée d'un dispositif stéréotaxique destiné à coopérer avec la tête du patient.

Un exemple de réalisation de l'invention va maintenant être décrit en regard des dessins annexés, sur lesquels :
- la Figure 1 est une vue d'ensemble schématique, en élévation latérale et en coupe, d'un appareil de traitement conforme à l'invention, en position d'attente;
- la Figure 2 représente de façon analogue l'appareil en cours de traitement;
- la Figure 3 est une vue de profil de l'ensemble sources-collimateur indiquant les positions possibles de la tête du patient;
- la Figure 4 est une vue de face de l'objet de la Figure 3;
- la Figure 5 représente en coupe longitudinale, à plus grande échelle, la région de l'ensemble sources-collimateur associée à une source;
- la Figure 6 illustre en perspective l'ensemble des canaux associés à une source;
- la Figure 7 est une vue prise suivant la flèche VII de la Figure 5; et
- la Figure 8 illustre par un diagramme la répartition du flux d'énergie au voisinage du point de focalisation.

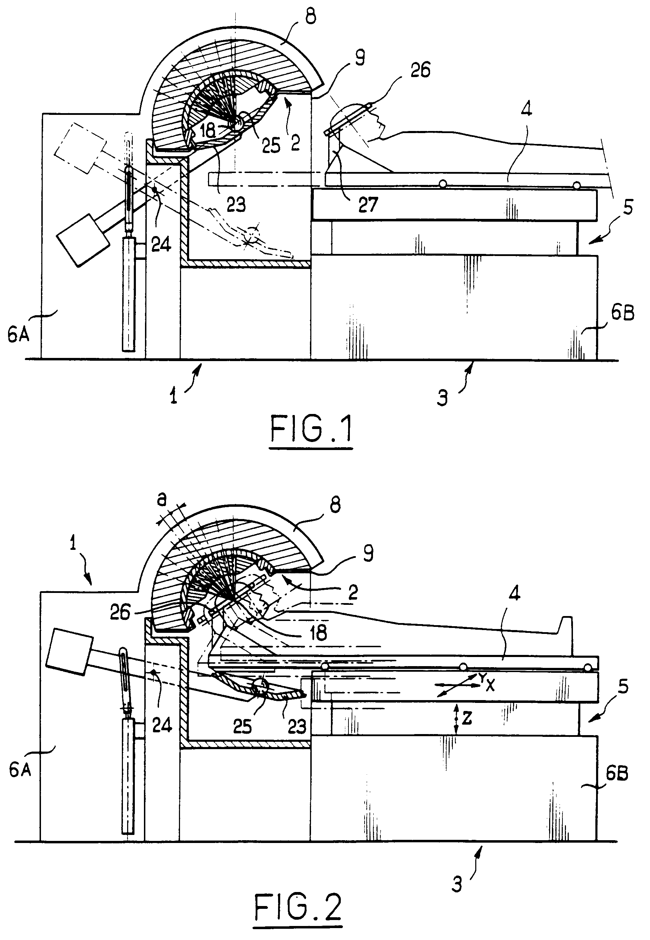

L'appareil de traitement de lésions cérébrales représenté aux Figures 1 et 2 est du type décrit dans le FR-A-2 672 220 précité. Il comprend un bâti principal fixe 1 dans la partie supérieure duquel est monté un ensemble sources-collimateur 2. A ce bâti est accolé un bâti secondaire fixe 3 qui supporte une table porte-patient 4, ceci par l'intermédiaire d'un dispositif motorisé 5 à commande numérique qui permet de déplacer la table parallèlement à elle-même suivant trois directions orthogonales X (qui est la direction longitudinale de la table), Y et Z (direction verticale). Les bâtis 1 et 3 contiennent par ailleurs des moyens électroniques de calcul de la commande, schématisés en 6A et 6B respectivement, appropriés pour assurer le fonctionnement qui sera décrit plus loin.

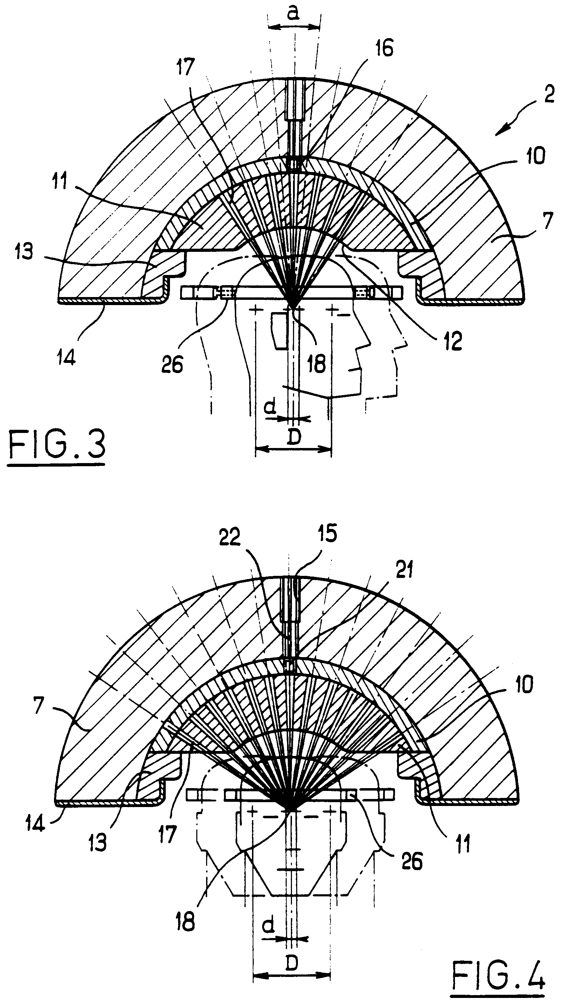

L'ensemble sources-collimateur 2, que l'on voit mieux aux Figures 3 et 4, a une forme générale sensiblement hémisphérique. Il comprend un blindage extérieur 7 de forte épaisseur, en plomb pur ou légèrement allié, dont la surface extérieure épouse avec un faible jeu radial la surface intérieure d'une coquille 8 du bâti 1. Cette coquille est fermée de tous côtés à l'exception d'une ouverture avant 9 juste suffisante pour laisser passer la table 4 portant un patient.

Dans le blindage 7 sont positionnés, d'une part un porte-sources 10 de faible épaisseur, et d'autre part, à l'intérieur de celui-ci, un collimateur 11 de forte épaisseur, ce dernier définissant une surface intérieure sphérique 12. Les organes 10 et 11 sont fixés dans le blindage au moyen d'une bague périphérique 13 elle-même maintenue par un flasque annulaire serti 14.

Dans sa région centrale en vue de profil (Figure 3), et sur une étendue angulaire plus importante en vue de face (Figure 4), l'ensemble 2 est traversé de part en part par un grand nombre de groupes de canaux radiaux. Chaque groupe est constitué d'un canal extérieur étagé 15 traversant le blindage, d'un canal cylindrique 16 traversant le porte-sources, et d'un faisceau de canaux 17 traversant le collimateur. Les axes de tous ces canaux convergent vers un point de focalisation 18, qui est le centre de la sphère.

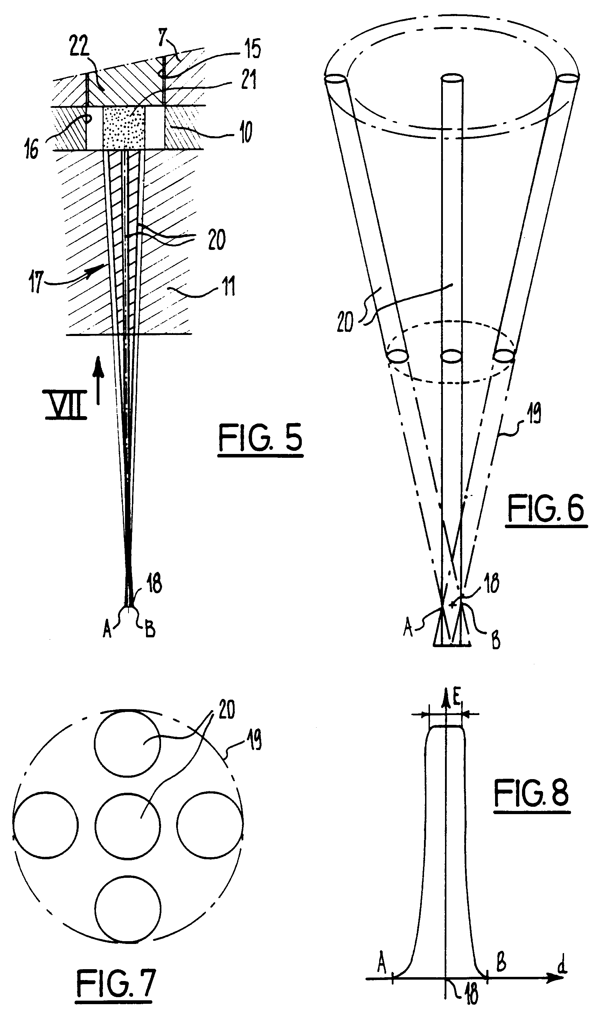

Comme représenté sur les Figures 5 à 7, chaque faisceau 17 s'inscrit dans une enveloppe fictive 19 de forme conique, dont le sommet est situé au point 18. La grande base et la petite base de l'enveloppe 19, au niveau de la surface extérieure et de la surface intérieure du collimateur 11, respectivement, ont des diamètres de l'ordre de 20 mm et de l'ordre de 10 mm. Le faisceau 17 est constitué d'un certain nombre de fins canaux cylindriques 20 dont tous les axes convergent vers le point 18. Le diamètre de chaque canal 20 est de l'ordre de 2 à 3 mm, et le nombre de canaux d'un faisceau est fonction de la place disponible dans l'enveloppe 19. Ce nombre est par exemple de cinq au moins, comme illustré sur la Figure 7.

La masse du collimateur 11 est constituée d'un matériau absorbant fortement les rayons gamma, notamment en plomb pur ou faiblement allié ou en uranium naturel ou appauvri en isotope 235, rendu inoxydable par dépôt d'une couche protectrice ou par alliage, notamment sur la paroi de chaque canal 20.

Une source de rayons gamma en césium 137, de forme cylindrique, dont le diamètre est à peu près égal au plus grand diamètre de l'enveloppe 19, est disposée dans le canal 16 et y est maintenue par un organe presseur 22 introduit et fixé de façon appropriée dans le canal 15 (Figures 3 et 4).

Une porte 23 est montée basculante sur le bâti 1 autour d'un axe horizontal et transversal 24, et porte un bloc 25 constitué d'un matériau absorbant fortement les rayons gamma, par exemple d'uranium.

La table 4 porte un dispositif stéréotaxique 26 supporté par des montants 27 fixés à la table.

On décrira maintenant le fonctionnement de l'appareil.

Une phase préalable d'investigation a permis de positionner la tête du patient dans le dispositif stéréotaxique 26 et de repérer dans l'espace la lésion à traiter.

Pour le traitement, la table 4 étant sortie du bâti 1 (Figure 1), on commence par repositionner la tête du patient de la même manière. La porte 23 est alors fermée et s'applique sur le pourtour de la bague 13, et le bloc 25 se trouve au point de focalisation 18 et absorbe les rayons gamma focalisés.

La porte 23 est ensuite ouverte, et la tête du patient est introduite sous le collimateur à travers l'ouverture 9, en faisant effectuer à la table un premier mouvement d'approche en X et Z puis un mouvement d'ajustement final par des mouvements en X et Y de l'ordre de ± 70 mm (référence D sur les Figures 3 et 4). Ceci amène la lésion, repérée grâce au dispositif 26, en coïncidence avec le point 18. La tête se trouve alors à une distance radiale notable de la surface intérieure 12 de l'ensemble 2, distance qui est très supérieure aux dimensions de la lésion à traiter.

Puis, pendant le traitement, le suivi du contour de la lésion est obtenu par des faibles mouvements en X, Y et Z de la table, de l'ordre de ± 10 mm, appelés mouvements de traitement (référence d sur les Figures 3 et 4). Le collimateur est simultanément animé d'un mouvement d'oscillation selon un angle a faible d'environ ± 5° autour de l'axe précité qui passe par le point de focalisation des rayons gamma (Figure 3).

Le mouvement tridimensionnel de la table 4 est très précis, du type utilisé dans certaines machines-outils à commande numérique. Il est commandé par ordinateur, à partir des données des imageries médicales obtenues par scanner, résonance magnétique ou autre. Il permet de traiter en une seule opération, à partir du seul réglage de départ, des lésions de formes quelconques avec une grande précision.

Le mouvement d'oscillation de l'ensemble 2 permet de ne pas léser les tissus sains situés entre lui-même et le point de focalisation, et il peut aussi permettre d'éviter tout contact avec les rayons gamma de zones particulièrement sensibles du cerveau.

Par ailleurs, chaque élément de surface de la source situé en regard d'un canal émet des rayonnements qui soit seront absorbés par la paroi de ce canal, soit passeront au point 18 ou très prés de celui-ci. En particulier, dans un plan perpendiculaire à l'axe central de l'enveloppe 20, tout le faisceau de rayons est concentré sur une distance AB très petite (Figures 5 et 6). De plus, comme représenté sur la Figure 8, où l'énergie cumulée E des rayons gamma est portée en ordonnées et la distance d au point 18 en abscisses, la quasi-totalité d'énergie des rayons gamma est concentrée dans la région qui entoure immédiatement le point focal 18, en formant un créneau presque rectangulaire, l'énergie étant pratiquement nulle aux points A et B. En d'autres termes, on obtient une tâche focale relativement étroite, ayant typiquement un diamètre de l'ordre de quelques mm, notamment de 2 à 4 mm, et dans laquelle est concentrée la quasi-totalité de l'énergie gamma.

## Revendications

1. Dispositif de traitement de lésions cérébrales par rayonnement gamma, comprenant un ensemble sources-collimateur (2) à peu près hémisphérique comportant un grand nombre de sources de rayons gamma (21) associées à des canaux (20) qui sont tous orientés vers un même point de focalisation (18), caractérisé en ce que chaque source (21) de rayons gamma est associée à un faisceau (17) de canaux (20) inscrit dans une enveloppe de révolution conique (19) dont le sommet est situé au point de focalisation (18).

2. Dispositif suivant la revendication 1, caractérisé en ce que chaque source (21) se trouve en regard de l'ouverture d'entrée de tous les canaux (20) du faisceau (17) qui lui est associé.

3. Dispositif suivant la revendication 1 ou 2, caractérisé en ce que tous les canaux (20) sont cylindriques.

4. Dispositif suivant l'une quelconque des revendications 1 à 3, caractérisé en ce que tous les canaux (20) ont sensiblement les mêmes dimensions.

5. Dispositif suivant l'une quelconque des revendications 1 à 4, caractérisé en ce que chaque faisceau (17) comporte au moins cinq canaux (20).

6. Appareil de traitement de lésions cérébrales par rayonnement gamma, comprenant un dispositif suivant l'une quelconque des revendications 1 à 5, et une table porte-patient mobile (4) équipée d'un dispositif stéréotaxique (26) destiné à coopérer avec la tête du patient.

## Claims

1. Device for the treatment of cerebral lesions using gamma radiation, including a sources-collimator assembly (2) approximately hemispherical in shape containing a large number of gamma ray sources (21) associated with channels (20) which are all orientated towards the same focusing point (18), characterized in that each gamma ray source (21) is associated with a beam (17) of channels (20) set in a conical revolution envelope (19) the apex of which is situated at the focusing point (18).

2. Device according to claim 1, characterized in that each source (21) is located opposite the inlet opening of all the beam (17) channels (20) with which it is associated.

3. Device according to claim 1 or 2, characterized in that all the channels (20) are cylindrical.

4. Device according to any one of claims 1 to 3, characterized in that all the channels (20) have approximately the same dimensions.

5. Device according to any one of claims 1 to 4, characterized in that each beam (17) comprises at least five channels (20).

6. Treatment apparatus for cerebral lesions using gamma radiation, including a device according to any one of claims 1 to 5 and a mobile patient-carrying table equipped with a stereotaxic device (26) intended to cooperate with the patient's head.

## Patentansprüche

1. Vorrichtung zur Behandlung von Gehirnschädigungen durch Gammastrahlung, das eine im wesentlichen halbkugelförmige Einheit (2) aus Quellen und Kollimator aufweist, die eine Vielzahl von Gammastrahlenquellen (21) aufweist, zu denen Kanäle (20) gehören, die alle in Richtung auf ein und denselben Brennpunkt (18) ausgerichtet sind, dadurch gekennzeichnet, daß zu jeder Gammastrahlenquelle (21) ein Bündel (17) von Kanälen (20) gehört, das auf einer konischen Drehumhüllenden (19) angeordnet ist, deren Scheitel sich am Brennpunkt (18) befindet.

2. Vorrichtung nach Anspruch 1, dadurch gekennzeichnet, daß jede Quelle (21) sich gegenüber der Eintrittsöffnung aller Kanäle (20) des Bündels (17) befindet, das zu ihr gehört.

3. Vorrichtung nach Anspruch 1 oder 2, dadurch gekennzeichnet, daß alle Kanäle (20) zylindrisch sind.

4. Vorrichtung nach einem der Ansprüche 1 bis 3, dadurch gekennzeichnet, daß alle Kanäle (20) im wesentlichen die gleichen Abmessungen haben.

5. Vorrichtung nach einem der Ansprüche 1 bis 4, dadurch gekennzeichnet, daß jedes Bündel (17) mindestens fünf Kanäle (20) umfaßt.

6. Gerät zur Behandlung von Gehirnschädigungen durch Gammastrahlung, das eine Vorrichtung nach einem der Ansprüche 1 bis 5 sowie einen beweglichen Patiententragetisch (4) umfaßt, der mit einem stereotaktischen Gerät (26) ausgerüstet ist, das vorgesehen ist, mit dem Kopf des Patienten zusammenzuwirken.
